# EUROPEAN PATENT SPECIFICATION

(11) **EP 4 444 410 B1**
(45) Date of publication and mention of the grant of the patent: **01.04.2026**
(21) Application number: 22817628.5
(22) Date of filing: 11.11.2022
(51) Int. Cl.: A61N 1/372, A61B 5/00, G16H 10/60, H04W 4/80

(54) **SYSTEM AND METHOD FOR OPERATING AN ACTIVE MEDICAL IMPLANT**
SYSTEM UND VERFAHREN ZUM BETRIEB EINES AKTIVEN MEDIZINISCHEN IMPLANTATS
SYSTÈME ET PROCÉDÉ DE FONCTIONNEMENT D'UN IMPLANT MÉDICAL ACTIF

(30) Priority: 06.12.2021 EP 21212421
(43) Date of publication of application: 16.10.2024
(73) Proprietor: BIOTRONIK SE & Co. KG, 12359 Berlin (DE)
(72) Inventor: MUELLER, Jens, 14195 Berlin (DE); DOERR, Thomas, 12437 Berlin (DE); SELENT, Miro, 14558 Nuthetal (DE)
(74) Representative: Biotronik Corporate Services SE
(86) International application number: PCT/EP2022/081623
(87) International publication number: WO 2023/104428

(56) References cited:
- US-A1- 2002 082 665
- US-A1- 2014 273 824
- US-A1- 2015 341 785
- US-A1- 2016 330 573
- US-A1- 2020 094 062
- US-A1- 2020 197 711

## Description

Embodiments of the present disclosure relate to a system for operating an active medical implant, a method for operating an active medical implant, and a machine-readable medium for executing the method. Embodiments of the present disclosure relate more particularly to an emergency function of an active medical implant.

Medical implants are widely used to replace, support and/or enhance biological structures of patients. Examples of medical implants include, but are not limited to, cardiovascular medical devices such as artificial hearts, artificial heart valves, implantable cardioverter-defibrillators, cardiac pacemakers, and coronary stents. Some medical implants are configured to record data, such as patient health data. However, retrieving the recorded data from the medical implant often requires specialized external equipment, Internet access, and/or trained personnel. As a result, retrieving the recorded data from the medical implant can be difficult, time consuming, and/or costly.

US2014/273824, US2020/197711 and US2002/082665 disclose the preamble of independent claims 1 and 13.

In light of the above, a system for operating an active medical implant, a method for operating an active medical implant, and a machine-readable medium for executing the method that overcome at least some of the problems in the art are beneficial.

It is an object of the present disclosure to provide a system for operating an active medical implant, a method for operating an active medical implant, and a machine-readable medium for executing the method that can facilitate retrieval of data from an active medical implant, particularly in emergency situations.

The objects are solved by the features of the independent claims. Preferred embodiments are defined in the dependent claims.

According to an independent aspect of the present disclosure, a system for operating an active medical implant is provided. The system includes an active medical implant configured to store information related to a patient's health status in a memory of the active medical implant, wherein the active medical implant has a first short-range communication module; and a mobile terminal having a second short-range communication module configured to directly communicate with the first short-range communication module of the active medical implant via a communication link. The active medical implant is configured to transmit the information related to the patient's health status to the mobile terminal via the communication link.

According to some embodiments, which can be combined with other embodiments described herein, the mobile terminal is configured to execute an application software (App). The application software may be configured to execute at least some of the functionalities of the mobile terminal described herein, such as receiving user inputs and/or outputting information, particularly the information related to the patient's health status received from the active medical implant.

According to some embodiments, which can be combined with other embodiments described herein, the mobile terminal is configured to send an information transmission request to the active medical implant to request the transmission of the information related to the patient's health status. The active medical implant may be configured to transmit the information related to the patient's health status upon receipt of the information transmission request.

According to some embodiments, which can be combined with other embodiments described herein, the first short-range communication module and the second short-range communication module are configured for Near Field Communication (NFC) and/or Bluetooth (BLE).

According to some embodiments, which can be combined with other embodiments described herein, the first short-range communication module and the second short-range communication module are configured to establish the communication link using a pairing mode.

Preferably, a number of pairing attempts to establish the communication link is equal to, or less than, a predetermined number.

Additionally, or alternatively, a duration of the pairing mode is equal to, or less than, a predetermined duration.

The first short-range communication module of the active medical implant is switchable by means of an external magnet device. The term "switchable" means that the first short-range communication module of the active medical implant can be turned on and turned off by means of the external magnet device.

According to some embodiments, which can be combined with other embodiments described herein, the active medical implant is configured to detect at least one predetermined situation related to the patient's health.

Preferably, the active medical implant is configured to store the information related to the patient's health status upon detection of the at least one predetermined situation.

Preferably, the at least one predetermined situation is selected from the group including (or consisting of) an emergency situation and a therapy situation or therapy procedure provided by the active medical implant.

According to some embodiments, which can be combined with other embodiments described herein, the information related to the patient's health status include one or more of the following:
- technical status information of the active medical implant; and/or
- medical alarms and/or technical alarms; and/or
- medical indications; and/or
- therapy parameters; and/or
- patient information; and/or
- physician information.

According to some embodiments, which can be combined with other embodiments described herein, the active medical implant is configured to encrypt the information related to the patient's health status before transmission thereof to the mobile terminal. The mobile terminal may be configured to decrypt the information related to the patient's health status which have been received from the active medical implant.

According to some embodiments, which can be combined with other embodiments described herein, the memory in which the information related to the patient's health status are stored is a dedicated memory which is logically and/or physically separate from other memories of the active medical implant.

According to some embodiments, which can be combined with other embodiments described herein, the active medical implant includes a processor module configured to control the storing of the information related to the patient's health status and/or the transmitting of the information related to the patient's health status to the mobile terminal.

Preferably, the processor module is a dedicated processor module which is logically and/or physically separate from other processor modules of the active medical implant.

According to some embodiments, which can be combined with other embodiments described herein, the communication link between the first short-range communication module and the second short-range communication module is a dedicated communication link which is logically separate from other communication links of the active medical implant.

According to some embodiments, which can be combined with other embodiments described herein, the active medical implant is configured to receive and execute at least one predefined command from the mobile terminal.

Preferably, the at least one predefined command is a command of a subset of commands executable by the active medical implant.

Preferably, the at least one predefined command is selected from the group including (or consisting of) a command to deactivate at least one therapy function of the active medical implant, a command to entirely deactivate the active medical implant, and a command to put the active medical implant in a predetermined mode of operation.

According to some embodiments, which can be combined with other embodiments described herein, the mobile terminal is selected from the group including (or consisting of) a smartphone, a personal digital assistant, a tablet, and a notebook.

Preferably, the mobile terminal is a mobile terminal of medical personnel, such as a mobile terminal of a physician.

According to another independent aspect of the present disclosure, a method for operating an active medical implant is provided. The method includes storing information related to a patient's health status in a memory of the active medical implant; sending, by a mobile terminal, an information transmission request to the active medical implant requesting a transmission of the information related to the patient's health status from the active medical implant to the mobile terminal, wherein the information transmission request is sent via a direct communication link between a first short-range communication module of the active medical implant and a second short-range communication module of the mobile terminal; and transmitting, by the active medical implant, the information related to the patient's health status to the mobile terminal via the communication link.

Embodiments are also directed at systems/devices for carrying out the disclosed methods and include system/device aspects for performing each described method aspect. These method aspects may be performed by way of hardware components, a computer programmed by appropriate software, by any combination of the two or in any other manner. Furthermore, embodiments according to the invention are also directed at methods for operating the described device/system. It includes method aspects for carrying out every function of the device/system.

According to another independent aspect of the present disclosure, a machine-readable medium is provided. The machine-readable medium includes instructions executable by one or more processors to implement the method for operating an active medical implant of the embodiments of the present disclosure.

The (e.g. non-transitory) machine readable medium may include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium may be used to tangibly retain computer program instructions or code organized into one or more modules and written in any desired computer programming language. When executed by, for example, one or more processors such computer program code may implement one or more of the methods described herein.

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1: shows a schematic view of a system for operating an active medical implant according to embodiments of the present disclosure;
- FIG. 2: shows a schematic view of a system for operating an active medical implant according to further embodiments of the present disclosure; and
- FIG. 3: shows a flowchart of a method for operating an active medical implant according to embodiments of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Some medical implants are configured to record data, such as patient health data. However, retrieving the recorded data from the medical implant often requires specialized external equipment, Internet access, and/or trained personnel. As a result, retrieving the recorded data from the medical implant can be difficult, time consuming, and/or costly.

According to the embodiments of the present disclosure, the active medical implant stores information related to a patient's health status for later retrieval. In particular, the active medical implant may automatically generate and store emergency information for later retrieval. The mobile terminal may read the information from the active medical implant using short-range communication, such as a Near Field Communication (NFC) Standard or Bluetooth Low Energy (BLE) Standard. Optionally, the mobile terminal may perform a basic management of the active medical implant, such deactivating particular functions or even the whole active medical implant.

Accordingly, the information related to a patient's health status are easily accessible using, for example, an application (App) on the mobile terminal. In particular, the information can be accessed for instance in an emergency room by non-specialists, such as medical personnel. Accordingly, even non-specialists can detect and evaluate the condition of the active medical implant in emergency situations and optionally control/activate/deactivate basic functions such as shock delivery or therapy functions. The embodiments of the present disclosure are cost-saving, scalable, and thus applicable even in small, decentralized emergency rooms. The system of the present disclosure can be used even in areas without internet connection or 4G/5G network.

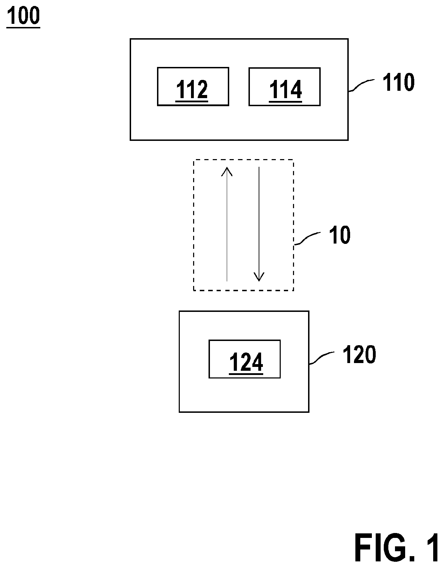

FIG. 1 shows a schematic view of a system 100 for operating an active medical implant 110 according to embodiments of the present disclosure.

The system 100 includes an active medical implant 110 and a mobile terminal 120, such as a tablet or other mobile device. In some embodiments, the mobile terminal 120 is a mobile terminal of medical personnel, such as a mobile terminal of a physician.

The mobile terminal 120 may be configured to execute an application software (App). The application software may be configured to execute at least some of the functionalities of the mobile terminal 120 described in the following, such as receiving user inputs and/or outputting information, particularly the information related to the patient's health status received from the active medical implant 110.

The active medical implant 110 is an electronic device which is implantable or implanted in a patient's body. The active medical implant 110 may perform diagnostic and/or therapeutic functions. In some implementations, the active medical implant 110 may be a cardiovascular medical device, such as an artificial heart, an artificial heart valve, an implantable cardioverter-defibrillator, a cardiac pacemaker, or a coronary stent. However, the present disclosure is not limited thereto, and other types of active medical implants can be used to implement the principles of the present disclosure.

The active medical implant 110 has a first short-range communication module 114. The mobile terminal 120 has a second short-range communication module 124. The first short-range communication module 114 and the second short-range communication module 124 are configured to directly communicate with each other via a communication link 10. Accordingly, an offline communication between the active medical implant 110 and the mobile terminal 120 is possible because no intermediate means such as servers and/or the Internet are needed to transfer data from the active medical implant 110 to the mobile terminal 120 and vice versa.

In some embodiments, the first short-range communication module 114 and the second short-range communication module 124 can be configured for Near Field Communication (NFC) and/or Bluetooth.

Near Field Communication refers to communication protocols for low-speed communication over a short distance, such as 4 cm or less. Bluetooth is a short-range wireless technology standard for exchanging data over short distances using UHF radio waves in the ISM bands from 2.402 GHz to 2.48 GHz.

Typically, the first short-range communication module 114 and the second short-range communication module 124 are configured to establish the communication link 10 using a pairing mode. The pairing mode refers to a state of the first short-range communication module 114 and the second short-range communication module 124 when they are ready to be paired.

In some embodiments, a number of pairing attempts to establish the communication link 10 is equal to, or less than, a predetermined number. This means that the paring mode will be terminated or the pairing attempts will be aborted after reaching the predetermined number, even if the pairing was not successful. Additionally, or alternatively, a duration of the pairing mode is equal to, or less than, a predetermined duration. This means that the paring mode will be terminated or the pairing attempts will be aborted after the predetermined duration has elapsed, even if the pairing was not successful. This limitation of the pairing mode can save energy and protect a battery of the active medical implant 110.

Pairing mode is where 114 and 124 are ready to pair. The predetermined number of pairing attempts takes effect precisely in this state. The predetermined duration is not only for the paring mode but also for the paired mode (state in which 114 and 124 are connected and are no longer in pairing mode). If a connection has been successfully established, the implant must be able to actively decide after a previously configured time X whether to interrupt the connection so that the battery is not depleted.

According to some implementations, the communication link 10 between the first short-range communication module 114 and the second short-range communication module 124 is a dedicated communication link which is logically separated from other communication links of the active medical implant 110. Examples of other communication links which can be established by the active medical implant 110 with external means include, but are not limited to, communication links for programming and/or interrogating the active medical implant 110, home monitoring communication links, etc.

In some embodiments, the first short-range communication module 114 of the active medical implant 110 can be switchable by means of an external magnet device, such as a permanent magnet. Hereto, the active medical implant 110, particularly the first short-range communication module 114, may include a magnetic switch. The term "switchable" means that the first short-range communication module 114 of the active medical implant 110 can be turned on and turned off by means of the external magnet device.

Accordingly, the active medical implant 110 may only connect to the mobile terminal 120 by placing a magnet on the patient's skin in the immediate vicinity of the active medical implant 110. The magnetic switch in the active medical implant 110 activates the communication interface, i.e., the communication link 10, from the outside and puts the active medical implant 110 into pairing mode. The magnetic activation ensures that no mobile terminal connects to the active medical implant 110 unintentionally without physical contact with the patient.

The active medical implant 110 includes the memory 112 configured to store information related to a patient's health status. For example, the active medical implant 110 may be configured to automatically obtain and store the information related to the patient's health status, for instance, upon detection of at least one predetermined situation related to the patient's health.

The at least one predetermined situation may include an emergency situation, such as the occurrence of a traumatic event. Exemplary traumatic events include, but are not limited to, a fall and a car accident.

In some embodiments, the active medical implant 110 may include at least one sensor configured to detect the emergency situation, such as a fall or a car accident. In particular, the at least one sensor may be configured to provide detection data suitable to derive the emergency situation therefrom. For example, the at least one sensor may include an acceleration sensor and/or a force sensor.

Additionally, or alternatively, the at least one predetermined situation may relate to a diagnostic and/or therapeutic functionality of the active medical implant 110. For example, the recording of the information related to the patient's health status may be started in the event of syncope in bradycardia, ventricular fibrillation in tachycardia, etc.

The information related to the patient's health status, which are stored in the memory 112 upon detection of the at least one predetermined situation, may include one or more of the following:
- technical status information of the active medical implant (e.g., operating status); and/or
- medical alarms and/or technical alarms; and/or
- medical indications; and/or
- therapy parameters (e.g., stimulation mode, shock on/off); and/or
- patient information (e.g., name, date of birth, age, sex, etc.); and/or
- physician information (e.g., name or other identification of the physician).

However, the present disclosure is not limited to the above examples and the information related to the patient's health status may include other information obtainable by the active medical implant 110.

According to some embodiments, the memory 112 in which the information related to the patient's health status are stored is a dedicated memory which is logically and/or physically separate from other memories of the active medical implant 110. Examples of other memories of the active medical implant 110 include, but are not limited to, memories used in therapeutic and diagnostic functionalities of the active medical implant 110, such as memories used in a pacemaker functionality.

The mobile terminal 120 may be configured to send an information transmission request or command to the active medical implant 110 via the communication link 10 to request the transmission of the information related to the patient's health status stored in the memory 112. The active medical implant 110 may be configured to transmit the information related to the patient's health status upon receipt of the information transmission request or command from the mobile terminal 120.

In some implementations, the active medical implant 110 may be configured to encrypt the information related to the patient's health status before transmission thereof to the mobile terminal 120. For example, in addition to the security mechanisms of the Near Field Communication standard and/or Bluetooth Low Energy standard, the transmitted information is optionally protected for example at the business layer level by further procedures.

The active medical implant 110 may be configured to encrypt the information related to the patient's health status before the information is stored in the memory 112. In other words, encrypted information is stored in the memory 112. Alternatively, the information may be stored in the memory 112 unencrypted and may be encrypted just before the transmission of the information to the mobile terminal 120.

The mobile terminal 120 may be configured to decrypt the information related to the patient's health status which have been received from the active medical implant 110 via the communication link 10.

According to some embodiments, the active medical implant 110 includes a processor module configured to control the storing of the information related to the patient's health status and/or the transmitting of the information related to the patient's health status to the mobile terminal 120. The processor module may be a dedicated processor module which is logically and/or physically separate from other processor modules of the active medical implant 110. Examples of other processor modules of the active medical implant 110 include, but are not limited to, processor modules configured to perform therapeutic and/or diagnostic functionalities of the active medical implant 110.

Due to the separation of the process for reading out the memory 112 from the rest of the active medical implant 110, the process for reading out the memory 112 has no effect on the diagnostic and/or therapeutic function of the active medical implant 110.

According to some embodiments, the active medical implant 110 may be configured to receive and execute at least one predefined command from the mobile terminal 120. The at least one predefined command may be a command of a subset of commands executable by the active medical implant 120. In other words, only a limited number of commands is accepted by the active medical implant 110 from the mobile terminal 120. Thereby, a safety of the active medical implant 110 can be improved because it can be avoided that untrained personnel changes for instance critical settings of the active medical implant 110.

The at least one predefined command may be a command to entirely deactivate the active medical implant or a command to put the active medical implant 110 in a predetermined mode of operation. For example, the at least one predefined command may be a command to disable shock delivery (e.g., for defibrillators), a command to set the active medical implant 110 to VVI mode (e.g., right ventricular sense, stimulate, inhibit), or a command to the active medical implant 110 to 000 mode (deactivate).

According to some embodiments, which can be combined with other embodiments described herein, the mobile terminal 120 includes a user interface. The user interface of the mobile terminal 120 may include a touch user interface and/or a voice user interface. For example, the user interface may include a touch screen configured to receive a touch input from a physician. Additionally, or alternatively, the user interface may be configured to receive a voice input from the physician.

The mobile terminal 120 may be configured to output the information received from the active medical implant 110. In some embodiments, the mobile terminal 120 may control the user interface to output the received information visually e.g. on a display and/or auditory e.g. using a loudspeaker.

In view of the foregoing, a direct communication between the active medical implant 110 and the mobile terminal 120 to read out and manage the active medical implant 110 is used. The direct communication between the active medical implant 110 and the mobile terminal 120 is advantageous because, in order to read out and manage the active medical implant 110, it is not necessary for the data to leave a protected environment (e.g., hospital, practice, etc.), as a locally operated mobile terminal e.g. of the clinical staff is used, and the data is also processed and displayed locally.

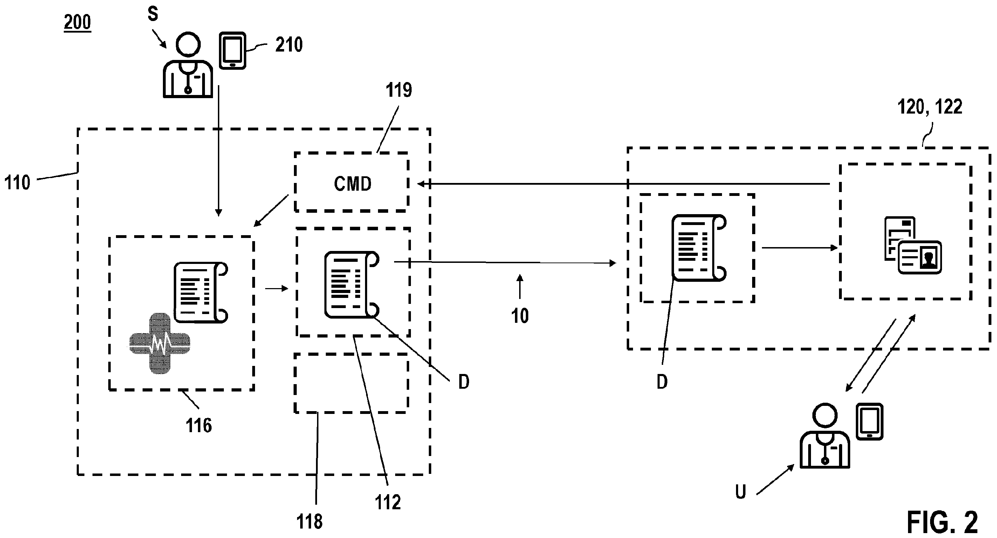

FIG. 2 shows a schematic view of a system 200 for operating an active medical implant 110 according to further embodiments of the present disclosure.

A specialist S may configure the active medical implant 110 e.g. during implantation thereof using a specialized external programming instance 210. The embodiments of the present disclosure, and in particular the mobile terminal, do not refer to such a configuration of the active medical implant 110 by a specialist and a specialized external programming instance 210. Instead, the embodiments of the present disclosure relate for instance to situations in which no specialist and no specialized external programming instance 210 is at hand.

The system 200 may be configured for an automatic generation of emergency information in the active medical implant 110 and the possibility of reading this emergency information later. In addition, the system 200 may be configured for a basic management of the active medical implant 110 by the mobile terminal 120, particularly an App 122 on the mobile terminal 120, using short-range communication. The reading and management may be performed in an emergency situation by non-specialists, such as medical staff in an emergency room.

The short-range communication between the active medical implant 110 and the mobile terminal 120 may implement the NFC standard or the BLE standard.

If the active medical implant 110 detects an emergency situation, the active medical implant 110 automatically stores at least some of the following information in a specially designated memory area, namely the memory 112 ("emergency memory"):
- patient information (e.g., first name, last name, date of birth)
- attending physician
- indication
- technical condition of the active medical implant
- medical and technical alarms
- therapy setting (e.g., pacing mode, shock on/off info for defibrillators)

A clinical user U can read the memory 112 from of the active medical implant 110 with the mobile terminal 120. This can be done by placing a magnet on the skin in close proximity to the active medical implant 110. The magnet activates a magnetic switch in the active medical implant 110, which signals a processing unit 118 in the active medical implant 110 to activate the short-range communication link 10.

An App 122 is locally operated on the mobile terminal 120. The App 122 establishes a connection with the active medical implant 110 via the communication link 10 and sends a command to the active medical implant 110 with the instruction to send the data D from the memory 112.

In the active medical implant 110, the memory 112, the communication link 10 and the processing module 118 for reading the memory 112 may be logically separated from the diagnostic-therapeutic part 116 (communication units and processing units are not shown).

For data transmission from the active medical implant 110 to the mobile terminal 120, the data D may be protected from data theft using, for example, symmetric encryption (e.g., AES, RC5). The App 122 decrypts and processes the queried data D locally on the mobile terminal 120 and displays the data D to the clinical user U.

In some implementations, if no data has been created and stored at the time the emergency memory 112 is queried, this can be done ad-hoc upon receipt of the command from the mobile terminal 120.

In some embodiments, the App 122 may provide a function to deactivate e.g. a shock function and/or an entire pacing function. After selecting one of these functions, the corresponding command is sent to the active medical implant 110 and processed there. A syntactic and semantic check of the command in a separate processing module (e.g., the processing module 118 or another processing module 119) may serve as a firewall.

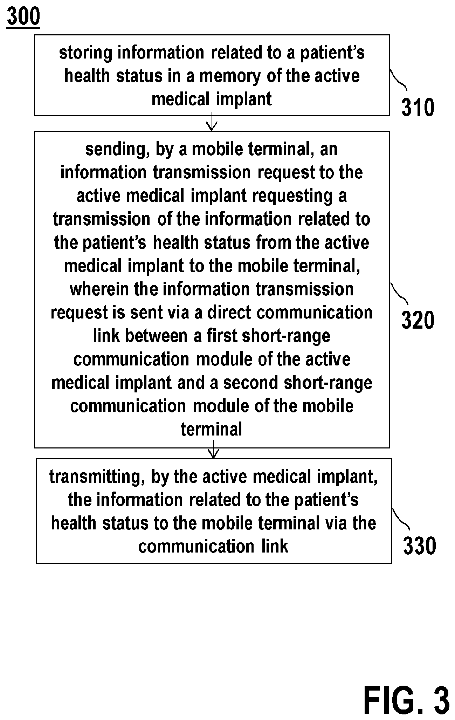

FIG. 3 shows a flowchart of a method 300 for operating an active medical implant according to embodiments of the present disclosure.

The method 300 includes in block 310 a storing of information related to a patient's health status in a memory of the active medical implant; in block 320 a sending, by a mobile terminal, of an information transmission request to the active medical implant requesting a transmission of the information related to the patient's health status from the active medical implant to the mobile terminal, wherein the information transmission request is sent via a direct communication link between a first short-range communication module of the active medical implant and a second short-range communication module of the mobile terminal; and in block 330 a transmitting, by the active medical implant, of the information related to the patient's health status to the mobile terminal via the communication link.

According to the embodiments of the present disclosure, the active medical implant stores information related to a patient's health status for later retrieval. In particular, the active medical implant may automatically generate and store emergency information for later retrieval. The mobile terminal may read the information from the active medical implant using short-range communication, such as a Near Field Communication (NFC) Standard or Bluetooth Low Energy (BLE) Standard. Optionally, the mobile terminal may perform a basic management of the active medical implant, such deactivating particular functions or even the whole active medical implant.

Accordingly, the information related to a patient's health status are easily accessible using, for example, an application (App) on the mobile terminal. In particular, the information can be accessed for instance in an emergency room by non-specialists, such as medical personnel. Accordingly, even non-specialists can detect and evaluate the condition of the active medical implant in emergency situations and optionally deactivate basic functions such as shock delivery or therapy functions. The embodiments of the present disclosure are cost-saving, scalable, and thus applicable even in small, decentralized emergency rooms. The system of the present disclosure can be used even in areas without internet connection or 4G/5G network.

While the foregoing is directed to embodiments of the disclosure, other and further embodiments of the disclosure may be devised without departing from the basic scope thereof, and the scope thereof is determined by the claims that follow.

## Claims

1. A system (100, 200) for operating an active medical implant (110), comprising:
an active medical implant (110) configured to store information (D) related to a patient's health status in a memory (112) of the active medical implant (110), wherein the active medical implant (110) has a first short-range communication module (114); and
a mobile terminal (120) having a second short-range communication module (124) configured to directly communicate with the first short-range communication module (114) of the active medical implant (110) via a communication link (10),
wherein the active medical implant (110) is configured to transmit the information (D) related to the patient's health status to the mobile terminal (120) via the communication link (10);
**characterised in that** the first short-range communication module (114) of the active medical implant (110) is switchable by means of an external magnet device.

2. The system (100, 200) of claim 1, wherein the mobile terminal (120) is configured to send an information transmission request to the active medical implant (110) to request the transmission of the information (D) related to the patient's health status, and wherein the active medical implant (110) is configured to transmit the information (D) related to the patient's health status upon receipt of the information transmission request.

3. The system (100, 200) of claim 1 or 2, wherein the first short-range communication module (114) and the second short-range communication module (124) are configured for at least one of Near Field Communication and Bluetooth.

4. The system (100, 200) of any one of claims 1 to 3, wherein the first short-range communication module (114) and the second short-range communication module (124) are configured to establish the communication link (10) using a pairing mode, wherein a number of pairing attempts to establish the communication link (10) is equal to, or less than, a predetermined number, and/or a duration of the pairing mode is equal to, or less than, a predetermined duration.

5. The system (100, 200) of any one of claims 1 to 4, wherein the active medical implant (110) is configured to detect at least one predetermined situation related to the patient's health, and wherein the active medical implant (110) is configured to store the information (D) related to the patient's health status upon detection of the at least one predetermined situation.

6. The system (100, 200) of any one of claims 1 to 5, wherein the information (D) related to the patient's health status include one or more of the following:
- technical status information of the active medical implant (110);
- medical alarms and/or technical alarms;
- medical indications;
- therapy parameters;
- patient information;
- physician information;

7. The system (100, 200) of any one of claims 1 to 6, wherein the active medical implant (110) is configured to encrypt the information (D) related to the patient's health status before transmission thereof to the mobile terminal (120), and wherein the mobile terminal (120) is configured to decrypt the information (D) related to the patient's health status which have been received from the active medical implant (110).

8. The system (100, 200) of any one of claims 1 to 7, wherein the memory (112) in which the information (D) related to the patient's health status are stored is a dedicated memory which is logically and/or physically separate from other memories of the active medical implant (110).

9. The system (100, 200) of any one of claims 1 to 8, wherein the active medical implant (110) includes a processor module (118) configured to control the storing of the information (D) related to the patient's health status and/or the transmitting of the information (D) related to the patient's health status to the mobile terminal (120), wherein the processor module (118) is a dedicated processor module which is logically and/or physically separate from other processor modules of the active medical implant (110).

10. The system (100, 200) of any one of claims 1 to 9, wherein the communication link (10) between the first short-range communication module (114) and the second short-range communication module (124) is a dedicated communication link which is logically separate from other communication links of the active medical implant (110).

11. The system (100, 200) of any one of claims 1 to 10, wherein active medical implant (110) is configured to receive and execute at least one predefined command from the mobile terminal (120), wherein the at least one predefined command is a command of a subset of commands executable by the active medical implant (110), in particular wherein the at least one predefined command is selected from the group consisting of:
- a command to deactivate at least one therapy function of the active medical implant (110);
- a command to entirely deactivate the active medical implant (110); and
- a command to put the active medical implant (110) in a predetermined mode of operation.

12. The system (100, 200) of any one of claims 1 to 11, wherein the mobile terminal (120) is selected from the group consisting of a smartphone, a personal digital assistant, a tablet, and a notebook.

13. A method (300) for operating an active medical implant, comprising:
storing (310) information related to a patient's health status in a memory of the active medical implant;
sending (320), by a mobile terminal, an information transmission request to the active medical implant requesting a transmission of the information related to the patient's health status from the active medical implant to the mobile terminal, wherein the information transmission request is sent via a direct communication link between a first short-range communication module of the active medical implant and a second short-range communication module of the mobile terminal; and
transmitting (330), by the active medical implant, the information related to the patient's health status to the mobile terminal via the communication link;
**characterised in that**
the first short-range communication module (114) of the active medical implant (110) is switchable by means of an external magnet device.

14. A machine-readable medium, comprising instructions executable by processors to implement the method (300) for operating an active medical implant of claim 13.

## Patentansprüche

1. Ein System (100, 200) zum Betreiben eines aktiven medizinischen Implantats (110), umfassend:
ein aktives medizinisches Implantat (110), das so konfiguriert ist, dass es Informationen (D) über den Gesundheitszustand eines Patienten in einem Speicher (112) des aktiven medizinischen Implantats (110) speichert, wobei das aktive medizinische Implantat (110) ein erstes Kurzstreckenkommunikationsmodul (114) aufweist; und
ein mobiles Endgerät (120) mit einem zweiten Kurzstrecken-Kommunikationsmodul (124), das so konfiguriert ist, dass es über eine Kommunikationsverbindung (10) direkt mit dem ersten Kurzstrecken-Kommunikationsmodul (114) des aktiven medizinischen Implantats (110) kommuniziert,
wobei das aktive medizinische Implantat (110) so konfiguriert ist, dass es die Informationen (D) über den Gesundheitszustand des Patienten über die Kommunikationsverbindung (10) an das mobile Endgerät (120) überträgt;
**dadurch gekennzeichnet, dass** das erste Kurzstrecken-Kommunikationsmodul (114) des aktiven medizinischen Implantats (110) mittels einer externen Magnetvorrichtung umschaltbar ist.

2. Das System (100, 200) nach Anspruch 1, wobei das mobile Endgerät (120) so konfiguriert ist, dass es eine Informationsübertragungsanforderung an das aktive medizinische Implantat (110) sendet, um die Übertragung der Informationen (D) über den Gesundheitszustand des Patienten anzufordern, und wobei das aktive medizinische Implantat (110) so konfiguriert ist, dass es die Informationen (D) über den Gesundheitszustand des Patienten nach Empfang der Informationsübertragungsanforderung überträgt.

3. Das System (100, 200) nach Anspruch 1 oder 2, wobei das erste Nahbereichskommunikationsmodul (114) und das zweite Nahbereichskommunikationsmodul (124) für mindestens eines von Nahfeldkommunikation und Bluetooth konfiguriert sind.

4. Das System (100, 200) nach einem der Ansprüche 1 bis 3, wobei das erste Kurzstreckenkommunikationsmodul (114) und das zweite Kurzstreckenkommunikationsmodul (124) so konfiguriert sind, dass sie die Kommunikationsverbindung (10) unter Verwendung eines Kopplungsmodus herstellen, wobei eine Anzahl von Kopplungsversuchen zum Herstellen der Kommunikationsverbindung (10) gleich oder kleiner als eine vorbestimmte Anzahl ist und/oder eine Dauer des Kopplungsmodus gleich oder kleiner als eine vorbestimmte Dauer ist.

5. Das System (100, 200) nach einem der Ansprüche 1 bis 4, wobei das aktive medizinische Implantat (110) so konfiguriert ist, dass es mindestens eine vorbestimmte Situation im Zusammenhang mit der Gesundheit des Patienten erkennt, und wobei das aktive medizinische Implantat (110) so konfiguriert ist, dass es die Informationen (D) im Zusammenhang mit dem Gesundheitszustand des Patienten bei Erkennung der mindestens einen vorbestimmten Situation speichert.

6. Das System (100, 200) nach einem der Ansprüche 1 bis 5, wobei die Informationen (D) in Bezug auf den Gesundheitszustand des Patienten eines oder mehrere der folgenden Elemente umfassen:
- technische Statusinformationen des aktiven medizinischen Implantats (110);
- medizinische Alarme und/oder technische Alarme;
- medizinische Indikationen;
- Therapieparameter;
- Patienteninformationen;
- Arztinformationen;

7. Das System (100, 200) nach einem der Ansprüche 1 bis 6, wobei das aktive medizinische Implantat (110) so konfiguriert ist, dass es die Informationen (D) über den Gesundheitszustand des Patienten vor der Übertragung an das mobile Endgerät (120) verschlüsselt, und wobei das mobile Endgerät (120) so konfiguriert ist, dass es die Informationen (D) zum Gesundheitszustand des Patienten, die vom aktiven medizinischen Implantat (110) empfangen wurden, entschlüsselt.

8. Das System (100, 200) nach einem der Ansprüche 1 bis 7, wobei der Speicher (112), in dem die Informationen (D) über den Gesundheitszustand des Patienten gespeichert sind, ein dedizierter Speicher ist, der logisch und/oder physisch von anderen Speichern des aktiven medizinischen Implantats (110) getrennt ist.

9. Das System (100, 200) nach einem der Ansprüche 1 bis 8, wobei das aktive medizinische Implantat (110) ein Prozessormodul (118) umfasst, das so konfiguriert ist, dass es das Speichern der Informationen (D) in Bezug auf den Gesundheitszustand des Patienten und/oder das Übertragen der Informationen (D) in Bezug auf den Gesundheitszustand des Patienten an das mobile Endgerät (120) zu steuern, wobei das Prozessormodul (118) ein dediziertes Prozessormodul ist, das logisch und/oder physisch von anderen Prozessormodulen des aktiven medizinischen Implantats (110) getrennt ist.

10. Das System (100, 200) nach einem der Ansprüche 1 bis 9, wobei die Kommunikationsverbindung (10) zwischen dem ersten Kurzstreckenkommunikationsmodul (114) und dem zweiten Kurzstreckenkommunikationsmodul (124) eine dedizierte Kommunikationsverbindung ist, die logisch von anderen Kommunikationsverbindungen des aktiven medizinischen Implantats (110) getrennt ist.

11. Das System (100, 200) nach einem der Ansprüche 1 bis 10, wobei das aktive medizinische Implantat (110) so konfiguriert ist, dass es mindestens einen vordefinierten Befehl vom mobilen Endgerät (120) empfängt und ausführt, wobei der mindestens eine vordefinierte Befehl ein Befehl einer Untergruppe von Befehlen ist, die vom aktiven medizinischen Implantat (110) ausgeführt werden können, wobei insbesondere der mindestens eine vordefinierte Befehl aus der Gruppe ausgewählt ist, die besteht aus:
- einem Befehl zum Deaktivieren mindestens einer Therapie-Funktion des aktiven medizinischen Implantats (110);
- einem Befehl zum vollständigen Deaktivieren des aktiven medizinischen Implantats (110); und
- einem Befehl zum Versetzen des aktiven medizinischen Implantats (110) in einen vorbestimmten Betriebsmodus.

12. Das System (100, 200) nach einem der Ansprüche 1 bis 11, wobei das mobile Endgerät (120) aus der Gruppe ausgewählt ist, die aus einem Smartphone, einem Personal Digital Assistant, einem Tablet und einem Notebook besteht.

13. Verfahren (300) zum Betreiben eines aktiven medizinischen Implantats, umfassend:
Speichern (310) von Informationen bezüglich des Gesundheitszustands eines Patienten in einem Speicher des aktiven medizinischen Implantats;
Senden (320) einer Informationsübertragungsanforderung durch ein mobiles Endgerät an das aktive medizinische Implantat, in der eine Übertragung der Informationen über den Gesundheitszustand des Patienten vom aktiven medizinischen Implantat an das mobile Endgerät angefordert wird, wobei die Informationsübertragungsanforderung über eine direkte Kommunikationsverbindung zwischen einem ersten Kurzstreckenkommunikationsmodul des aktiven medizinischen Implantats und einem zweiten Kurzstreckenkommunikationsmodul des mobilen Endgeräts gesendet wird; und
Übertragen (330) der Informationen über den Gesundheitszustand des Patienten durch das aktive medizinische Implantat an das mobile Endgerät über die Kommunikationsverbindung;
**dadurch gekennzeichnet, dass** das erste Kurzstrecken-Kommunikationsmodul (114) des aktiven medizinischen Implantats (110) mittels einer externen Magnetvorrichtung umschaltbar ist.

14. Ein maschinenlesbares Medium, das von Prozessoren ausführbare Befehle umfasst, um das Verfahren (300) zum Betreiben eines aktiven medizinischen Implantats gemäß Anspruch 13 zu implementieren.

## Revendications

1. Système (100, 200) destiné à faire fonctionner un implant médical actif (110), comprenant :
un implant médical actif (110) configuré pour stocker des informations (D) liées au statut de santé d'un patient dans une mémoire (112) de l'implant médical actif (110), dans lequel l'implant médical actif (110) a un premier module de communication à courte portée (114) ; et
un terminal mobile (120) ayant un second module de communication à courte portée (124) configuré pour communiquer directement avec le premier module de communication à courte portée (114) de l'implant médical actif (110) par l'intermédiaire d'une liaison de communication (10), dans lequel l'implant médical actif (110) est configuré pour transmettre les informations (D) liées au statut de santé du patient au terminal mobile (120) par l'intermédiaire de la liaison de communication (10) ;
**caractérisé en ce que**
le premier module de communication à courte portée (114) de l'implant médical actif (110) peut être commuté au moyen d'un dispositif d'aimant externe.

2. Système (100, 200) selon la revendication 1, dans lequel le terminal mobile (120) est configuré pour envoyer une requête de transmission d'informations à l'implant médical actif (110) pour exiger la transmission des informations (D) liées au statut de santé du patient, et dans lequel l'implant médical actif (110) est configuré pour transmettre les informations (D) liées au statut de santé du patient lors de la réception de la requête de transmission d'informations.

3. Système (100, 200) selon la revendication 1 ou 2, dans lequel le premier module de communication à courte portée (114) et le second module de communication à courte portée (124) sont configurés pour au moins une parmi une communication en champ proche et une communication Bluetooth.

4. Système (100, 200) selon l'une quelconque des revendications 1 à 3, dans lequel le premier module de communication à courte portée (114) et le second module de communication à courte portée (124) sont configurés pour établir la liaison de communication (10) en utilisant un mode d'appariement, dans lequel un nombre de tentatives d'appariement destinées à établir la liaison de communication (10) est inférieur ou égal à un nombre prédéterminé, et/ou une durée du mode d'appariement est inférieure ou égale à une durée prédéterminée.

5. Système (100, 200) selon l'une quelconque des revendications 1 à 4, dans lequel l'implant médical actif (110) est configuré pour détecter au moins une situation prédéterminée liée à la santé du patient, et dans lequel l'implant médical actif (110) est configuré pour stocker les informations (D) liées au statut de santé du patient lors de la détection de l'au moins une situation prédéterminée.

6. Système (100, 200) selon l'une quelconque des revendications 1 à 5, dans lequel les informations (D) liées au statut de santé du patient incluent une ou plusieurs des suivantes :
- informations de statut technique de l'implant médical actif (110) ;
- alarmes médicales et/ou alarmes techniques ;
- indications médicales ;
- paramètres thérapeutiques ;
- informations de patient ;
- informations de médecin ;

7. Système (100, 200) selon l'une quelconque des revendications 1 à 6, dans lequel l'implant médical actif (110) est configuré pour chiffrer les informations (D) liées au statut de santé du patient avant la transmission de celles-ci au terminal mobile (120), et dans lequel le terminal mobile (120) est configuré pour déchiffrer les informations (D) liées au statut de santé du patient qui ont été reçues de la part de l'implant médical actif (110).

8. Système (100, 200) selon l'une quelconque des revendications 1 à 7, dans lequel la mémoire (112) dans laquelle les informations (D) liées au statut de santé du patient sont stockées dans une mémoire dédiée qui est logiquement et/ou physiquement séparée des autres mémoires de l'implant médical actif (110).

9. Système (100, 200) selon l'une quelconque des revendications 1 à 8, dans lequel l'implant médical actif (110) inclut un module de processeur (118) configuré pour commander le stockage des informations (D) liées au statut de santé du patient et/ou à la transmission des informations (D) liées au statut de santé du patient au terminal mobile (120), dans lequel le module de processeur (118) est un module de processeur dédié qui est logiquement et/ou physiquement séparé des autres modules de processeur de l'implant médical actif (110).

10. Système (100, 200) selon l'une quelconque des revendications 1 à 9, dans lequel la liaison de communication (10) entre le premier module de communication à courte portée (114) et le second module de communication à courte portée (124) est une liaison de communication dédiée qui est logiquement séparée des autres liaisons de communication de l'implant médical actif (110).

11. Système (100, 200) selon l'une quelconque des revendications 1 à 10, dans lequel l'implant médical actif (110) est configuré pour recevoir et exécuter au moins un ordre prédéfini de la part du terminal mobile (120), dans lequel l'au moins un ordre prédéfini est un ordre d'un sous-ensemble d'ordres exécutables par l'implant médical actif (110), en particulier dans lequel l'au moins un ordre prédéfini est choisi dans le groupe constitué par :
- un ordre pour désactiver au moins une fonction thérapeutique de l'implant médical actif (110) ;
- un ordre pour désactiver entièrement l'implant médical actif (110) ; et
- un ordre pour mettre l'implant médical actif (110) dans un mode de fonctionnement prédéterminé.

12. Système (100, 200) selon l'une quelconque des revendications 1 à 11, dans lequel le terminal mobile (120) est choisi dans le groupe constitué par un smartphone, un assistant numérique personnel, une tablette et un portable.

13. Procédé (300) destiné à faire fonctionner un implant médical actif, comprenant les étapes consistant à :
stocker (310) des informations liées au statut de santé d'un patient dans une mémoire de l'implant médical actif ;
faire envoyer (320), par un terminal mobile, requête de transmission d'informations à l'implant médical actif exigeant une transmission des informations liées au statut de santé du patient de l'implant médical actif au terminal mobile, dans lequel la requête de transmission d'informations est envoyée par l'intermédiaire d'une liaison de communication directe entre un premier module de communication à courte portée de l'implant médical actif et un second module de communication à courte portée du terminal mobile ; et
faire transmettre (330), par l'implant médical actif, les informations liées au statut de santé du patient au terminal mobile par l'intermédiaire de la liaison de communication ;
**caractérisé en ce que**
le premier module de communication à courte portée (114) de l'implant médical actif (110) peut être commuté au moyen d'un dispositif d'aimant externe.

14. Support lisible par machine, comprenant des instructions exécutables par des processeurs pour mettre en œuvre le procédé (300) destiné à faire fonctionner un implant médical actif selon la revendication 13.
